Europäisches Patentamt

European Patent Office

Office européen des brevets

(11) Publication number: **0 459 641 A2**

(12) # EUROPEAN PATENT APPLICATION

(21) Application number: 91304173.7

(22) Date of filing: 09.05.91

(51) Int. Cl.[5]: **C10M 109/00, C10M 111/04, C07C 35/08, C07C 13/50, // (C10M111/04, 105:02, 105:16, 107:10)**

(30) Priority: 31.05.90 US 531172
06.06.90 US 534080

(43) Date of publication of application:
04.12.91 Bulletin 91/49

(84) Designated Contracting States:
BE DE FR GB IT

(71) Applicant: **TEXACO CHEMICAL COMPANY**
**3040 Post Oak Boulevard**
**Houston, Texas 77056 (US)**

(72) Inventor: **Sanderson, John Ronald**
**P.O. Box 587**
**Leander, Texas 78761 (US)**
Inventor: **Marquis, Edward Thomas**
**9004 Collinfield Drive**
**Austin, Texas 78758 (US)**

(74) Representative: **Brock, Peter William et al**
**URQUHART-DYKES & LORD 91 Wimpole**
**Street**
**London W1M 8AH (GB)**

(54) **Synthetic lubricant base stocks.**

(57)    Synthetic lubricant base stocks comprising reduced oligomers prepared from an olefin composition comprising a linear olefin having from 10 to 24 carbon atoms additionally contain an alkyl-substituted cyclohexanol or alkyl-substituted decahydronaphthalene, whose alkyl groups have 10 to 24 carbon atoms.
    These base stocks are prepared by contacting the olefin composition and phenol or naphthalene with an acidic montmorillonite clay and hydrogenating the resulting oligomer mixture.

EP 0 459 641 A2

The invention relates to synthetic lubricant base stocks, and more particularly to synthetic lubricant base stocks having a desirably high viscosity, or desirably low pour point.

Synthetic lubricants have been prepared from man-made base stocks to have uniform molecular structures and, therefore, well-defined properties that can be tailored to specific applications. Mineral oil base stocks, on the other hand, are prepared from crude oil and are complex mixtures of naturally occurring hydrocarbons. The greater uniformity of synthetic lubricants generally results in superior performance properties. For example, synthetic lubricants have excellent thermal stability. As automobile engines are reduced in size to save weight and fuel, they run at higher temperatures, and therefore require a more thermally stable oil. Because lubricants made from synthetic base stocks have such properties as excellent oxidative/thermal stability, very low volatility, and good viscosity indices over a wide range of temperatures, they offer better lubrication and permit longer drain intervals, with less oil vaporization loss between oil changes.

Synthetic base stocks may be prepared by oligomerizing internal and alpha-olefin monomers to form a mixture of dimers, trimers, tetramers, and pentamers, with minimal amounts of higher oligomers. The unsaturated oligomer products are then hydrogenated to improve their oxidative stability, with little change in other properties. The resulting synthetic base stocks have uniform isoparaffinic hydrocarbon structures, similar to high quality paraffinic mineral base stocks, but have the superior properties mentioned above, because of their greater uniformity.

Synthetic base stocks are produced in a broad range of viscosity grades. It is common practice to classify the base stocks by their viscosities, measured in centistokes (cSt) at 100°C. Those base stocks with viscosities less than or equal to 4 cSt are commonly referred to as "low viscosity" base stocks, whereas base stocks having a viscosity of 40 to 100 cSt are commonly referred to as "high viscosity" base stocks. Base stocks having a viscosity of 4 to 8 cSt are referred to as "medium viscosity" base stocks. The low viscosity base stocks generally are recommended for low temperature applications. Compositions for use at higher temperatures, such as motor oils, automatic transmission fluids, turbine lubricants, and other industrial lubricants, generally require higher viscosities, such as those provided by medium viscosity base stocks (i.e. 4 to 8 cSt grades). High viscosity base stocks are used in gear oils, and as blending stocks.

The viscosity of the base stocks is generally determined by the length of the oligomer molecules formed during the oligomerization reaction. The degree of oligomerization is affected by the catalyst and reaction conditions employed during the oligomerization reaction. The length of the carbon chain of the monomer starting material also has a direct influence on the properties of the oligomer products. Fluids prepared from short-chain monomers tend to have low pour points and moderately low viscosity indices, whereas fluids prepared from long-chain monomers tend to have moderately low pour points and higher viscosity indices. Oligomers prepared from long-chain monomers are generally more suitable for use as medium viscosity synthetic lubricant base stocks than those prepared from shorter-chain monomers.

One known approach to oligomerizing long-chain olefins to prepare synthetic lubricant base stocks is to contact the olefin with boron trifluoride, together with a promotor, at a reaction temperature sufficient to effect oligomerization of the olefin, for example as described in US-A-4400565, -4420646, -4420647 and -4434308. Boron trifluoride gas ($BF_3$) is, however, a pulmonary irritant, and breathing the gas, or fumes formed by hydration of the gas with atmospheric moisture, poses hazards preferably avoided. In addition, the disposal/neutralization of $BF_3$ raises environmental concerns.

Thus, a method for oligomerizing long-chain olefins using a non-hazardous, non-polluting catalyst would be a substantial improvement in the art.

Kuliev et al. (Institute of Petrochemical Processes of the Academy of Sciences of the Azerbaidzhan SSR, Azer. Neft. Khoz., 1983, No. 4, pages 40-43) attempted to prepare synthetic lubricants by oligomerizing long-chain ($C_9$-$C_{14}$) olefins using non-hazardous and non-polluting acidic clays comprising sulfuric and hydrochloric acid-activated bentonites from the Azerbaidzhan SSR. Kuliev et al. concluded, however, that "it was not possible to prepare viscous or high-viscosity oils by olefin polymerization over an aluminosilicate catalyst" and that "hydrogen redistribution reactions predominate with formation of aromatic hydrocarbon, coke, and paraffinic hydrocarbon". US-A-4531014, on the other hand, describes the use of Wyoming bentonite to oligomerize shorter-chain olefins, but this process, like that of Kuliev et al., does not make it possible to obtain a product high in dimer, trimer and tetramer, and low in disproportionation products.

Our prior Application (P23492) 91302130 shows that it is possible to prepare synthetic lubricant base stocks in good yield by oligomerizing long-chain olefins using certain acidic montmorillonite clay catalysts. It was found that a high conversion of long-chain olefin into dimer, trimer and tetramer may be obtained, with formation of very little concomitant hydrogen redistribution by-product, by using an acidic calcium montmorillonite clay having a moisture content up to 20 wt.%, a residual acidity of 3 to 30mg KOH/g (when titrated to a phenolphthalein end point), and a surface area of 300 $M^2$/g or more. In addition to being excellent catalysts, these clays are non-hazardous and non-polluting.

The viscosity of base stocks prepared in this manner may be improved by co-reacting long-chain linear olefins and naphthalene in the presence of these acidic montmorillonite catalysts. The resulting mixtures of oligomers and alkylated naphthalenes exhibit a viscosity substantially higher than that of base stocks comprising the olefin oligomers alone. Additionally, incorporating the naphthalene lowers the cost of producing the base stocks by replacing a portion of the more expensive long-chain olefin feed with naphthalene.

US-A-4604491 describes the alkylation of naphthalene with long-chain linear olefins in the presence of acidic montmorillonite clay catalysts to prepare synthetic lubricant base stocks comprising mixtures of mono-alkylated and polyalkylated naphthalenes. We have discovered, surprisingly, that mixtures of alkylated decahydrodronaphthalenes and olefin oligomers also provide synthetic lubricant base stocks having an improved viscosity, but that have a lower pour point than those obtained from mixtures containing alkylated naphthalenes.

We have also discovered that the viscosity of base stocks prepared in this manner may be improved by co-reacting long-chain linear olefins and phenol in the presence of these acidic montmorillonite catalysts. The resulting mixtures of oligomers and alkylated phenols exhibit a viscosity higher than that observed in base stocks comprising the olefin oligomers alone. The incorporation of a hydroxy group (via the phenol) also serves to increase the additive solubility of the resulting base stock. Moreover, incorporating the phenol lowers the cost of producing the base stocks by replacing a portion of the more expensive long-chain linear olefin feed with phenol. The oligomers and alkylated phenols are hydrogenated (to alkanes and alkylated cyclohexanols) to enhance their oxidative stability.

One embodiment of this invention provides a synthetic lubricant base stock comprising reduced oligomers prepared from an olefin composition comprising a linear olefin having from 10 to 24 carbon atoms, which additionally contains an alkyl-substituted cyclohexanol or alkyl-substituted decahydronaphthalene, whose alkyl groups have 10 to 24 carbon atoms.

The linear olefins comprise alpha-olefins, internal olefins or a mixture thereof. Additional comonomers may be present in the composition which is oligomerized. That composition may, for example contain up to 20 weight % of propylene or of 1,3-diisopropenyl benzene or alpha-methyl styrene. Alternatively, the linear olefins may be employed with a vinylidene olefin having 10 to 24 carbon atoms.

The synthetic lubricant base stock according to the invention may be prepared by contacting the olefin composition together with phenol or naphthalene, with an acidic montmorillonite clay and hydrogenating the resulting oligomer mixture.

Optionally the clay is activated with a Lewis acid.

The viscosity of these base stocks is improved over the value of the materials from olefins alone, when the starting materials comprise a mixture of long-chain linear olefin and phenol or naphthalene preferably from 1 to 40 wt. % of phenol or naphthalene (i.e. in a weight ratio of phenol or naphthalene to linear olefin of 1:99 to 2:3). More preferably, the mixture of long-chain linear olefin and phenol contains from 5 to 25 wt.% of phenol at naphthalene (i.e. in a weight ratio of 1:20 to 1:4). Co-reacting the phenol or naphthalene and the linear olefin feed produces a mixture of olefin oligomers and alkylated phenols or alkylated naphthalenes. The double bonds present in the oligomers and alkylated phenols or naphthalenes contained in the mixture are then hydrogenated, to obtain a mixture of reduced oligomers and alkylated cyclohexanols. Preferably, the resulting base stocks contain from 1 to 80 wt.%, more preferably 5 to 40 wt.% of alkylated or alkylated decahydronaphthalenes. The presence of the alkylated decahydronaphthalenes leads to a lower pour point, and hydrogenation provides a modest increase in viscosity as well as an increase in stability. Preferably, the resulting base stocks contain from 1 to 80 wt. %, more preferably 5 to 40 wt% of alkylated cyclohexanol or decahydronaphthalene.

Olefin monomer feed stocks useful in the present invention include compounds comprising (1) alpha-olefins having the formula $R''CH=CH_2$, where $R''$ is an alkyl radical of 8 to 22 carbon atoms, and (2) internal olefins having the formula $RCH=CHR'$, where $R$ and $R'$ are the same or different alkyl radicals of 1 to 20 carbon atoms, provided that the total number of carbon atoms in any one olefin is from 10 to 24, inclusive. A preferred range for the total number of carbon atoms in any one olefin molecule is 12 to 18, inclusive, with an especially preferred range being 13 to 16, inclusive. Mixture of internal and alpha-olefin may be used, as well as mixtures of olefins having different numbers of carbon atoms, provided that the total number of carbon atoms in any one olefin is within the range of 10 to 24, inclusive. The alpha and internal-olefins and phenol ornaphthalene useful in the present invention may be obtained by processes well-known to those skilled in the art and are commercially available. As in our above-mentioned prior Application (P23492) 91302130.9 the linear olefins may also be supplemented by propylene, diisopropenyl benzene or alpha-methylstyrene, or by vinylidene olefins.

Oligomerization of linear olefins may be represented by the following general equation:

$$\text{catalyst}$$

$$nC_mH_{2m} \text{--------} > C_{mn}H_{2mn}$$

where n represents moles of monomer and m represents the number of carbon atoms in the monomer. Thus, oligomerization of 1-decene may be represented as follows:

$$nC_{10}H_{20} \xrightarrow{\text{catalyst}} C_{10n}H_{20n}$$

The reactions occur sequentially. Initially, olefin monomer reacts with olefin monomer to form dimers. The dimers that are formed then react with additional olefin monomer to form trimers, and so on. This results in an oligomer product distribution that varies with reaction time. As the reaction time increases, the olefin monomer conversion increases, and the selectivities for the heavier oligomers increase. Generally, each resulting oligomer contains one double bond.

The mono-alkylation of phenol or naphthalene by a linear olefin may be represented as follows:

$$C_mH_{2m} + C_6H_5OH \xrightarrow{\hspace{1cm}\text{catalyst}\hspace{1cm}} C_6H_4OH(C_mH_{(2m+1)})$$

$$C_mH_{2m} + C_{10}H_8 \xrightarrow{\hspace{2cm}} C_{10}H_7(C_mH_{(2m+1)})$$

where m = the number of carbon atoms in the olefin monomer. Depending on the mole ratio of linear olefin to phenol or naphthalene, and on the reaction conditions, poly-alkylation of the phenol or naphthalene will also occur. The alkylation of phenol or naphthalene by linear olefin feed occurs concurrently with the oligomerization of the olefin feed. Thus, the co-reaction results in a mixture of oligomers (dimers, trimers, tetramers, etc) and alkylated phenols or alkylated naphthalenes including mono-, di-, and tri-alkylated phenols or naphthalenes. The number of carbon atoms in the alkyl groups of the alkylated phenols or naphthalenes will correspond to the number of carbon atoms in the linear olefin feed. Thus, the alkyl group of the alkylated phenols or naphthalenes will have from 10 to 24 carbon atoms.

The catalyst useful to effect these reactions are certain silica-alumina clays (aluminosilicates). Silica-alumina clays primarily are composed of silicon, aluminium, and oxygen, with minor amounts of magnesium and iron in some instances. Variations in the ratios of these constituents, and in their crystal lattice configurations, result in some fifty separate clays, each with its own characteristic properties.

One class of silica-alumina clays comprises smectite clays, which have a small particle size and unusual intercalation properties, giving them a high surface area. Smectites comprise layered sheets of octahedral sites between sheets of tetrahedral sites, where the distance between the layers can be adjusted by swelling, using an appropriate solvent. Three-layered sheet-type smectites include the montmorillonites used according to the invention. The montmorillonite structure may be represented by the following formula:

$$M_{x/n}^{n+} \cdot yH_2O(Al_{4-x}Mg_x)(Si_8)O_{20}(OH)_4$$

where M represents the interlamellar (balancing) cations, normally sodium or lithium; and x, y and n are numbers.

Montmorillonite clays may be acid-activated by such minerals as sulphuric acid and hydrochloric acid. Mineral acids activate montmorillonites by attacking and solubilizing structural cations in the octahedral layers. This opens up the clay structure and increases surface area. These acid-treated clays act as strong Bronsted acids. We have discovered that certain acid-treated montmorillonite clay catalysts are particularly effective for preparing synthetic lubricant base stocks in good yield by oligomerizing long-chain olefins. These preferred clays are acidic calcium montmorillonite clays having a moisture content of up to 20 wt.%, a residual acidity of 3 to 30mg KOH/g (when titrated to a phenolphthalein end point), and a surface area of 300 M²/g or more. Illustrative examples include Filtrol grade 24, having a moisture content of 12 wt.%, a residual acidity of 8.5 mg KOH/g and a surface area of 425 M²/g; Filtrol grade 124, having a moisture content of 2 wt%, residual acidity of 7.0 mg KOH/g, and a surface area of 400 M²/g; Filtrol grade 13, having a moisture content of 16 wt.%, a residual acidity of 15 mg KOH/g, and a surface area of 300 M²/g; Filtrol grade 113, having a moisture content of 4 wt.%, a residual acidity of 10 mg KOH/g, and a surface area of 300 M²/g; and Filtrol grade 224, having virtually no moisture, and having a residual acidity of 3.0 mg KOH/g, and a surface area of 350 M²/g

Preferably, the clay catalyst is activated by heat treatment before running the co-reactions. We have found that heat treatment of the catalyst before running an oligomerization reaction causes the catalyst to be more active and produce a higher olefin conversion. Additionally, clays heat treated in this manner are more stable, remaining active during a reaction over a longer period. The clays may be heat treated at temperatures of 50 to 400°C, with or without the use of a vacuum. A more preferred temperature range is from 50 to 300°C. Optionally, an inert gas may be used during heat treatment as well. Preferably, the clay should be heat treated under conditions and for a length of time which will reduce the water content of the clay to 1 wt.% or less.

The oligomerization and alkylation co-reactions may be carried out in either a stirred slurry reactor or in a fixed bed continuous flow reactor. The catalyst concentration should be sufficient to provide the desired catalytic effect. The temperatures at which the oligomerization and alkylation may be performed are generally from 50 to 300°C, with the preferred range being 150 to 180°C. The reactions may be run at pressures of from 0.1 to 7 MPa (0 to 1000 psig).

According to our prior Application (P23492) 91302130.9 an even higher conversion of olefin into oligomer may be obtained when the acidic calcium montmorillonite clays are treated with a Lewis acid such as aluminium nitrate before they are used as a catalyst. Additionally, the resulting oligomer products contain a greater percentage of trimer and higher oligomers.

Preferably, the clay is treated with aluminium nitrate before running the reaction. The clay can be added to a solution of 1 to 20 wt.%, preferably 10 wt% of aluminium nitrate in water. The ratio of clay to aluminium nitrate solution should be sufficient to provide a washed and dried catalyst having a concentration of aluminium nitrate at least sufficient to give the Friedel-Crafts effect, preferably 0.01 to 10 wt. % of aluminium nitrate, but not so much that it inhibits the acid sites of the clay. The clay should remain in the aluminium nitrate solution under agitation for a period sufficient to meet these requirements.

Advantageous results may be obtained when certain other Lewis Acid moderators, e.g. nickel chloride or zinc chloride, are employed in the place of aluminium nitrate.

Following the oligomerization and alkylation co-reactions, the alkylated phenols or naphthalenes, and the partially unsaturated oligomers, are completely reduced via catalytic hydrogenation. Surprisingly, hydrogenation of the alkylated naphthalenes to alkylated decahydronaphthalenes results in a drop in the pour point of the resulting synthetic lubricant, with little or no negative impact on other properties, such as viscosity index. Hydrogenation of the oligomers improves their thermal stability and helps prevent oxidative degradation during use of the mixture as a lubricant. The hydrogenation reaction for 1-decene oligomers may be represented as follows:

$$\text{catalyst}$$
$$C_{10n}H_{20n} + H_2 \text{ --------->} C_{10n}H_{(20n+2)}$$

where n represents moles of monomer used to form the oligomer. The hydrogenation of a mono-alkylated naphthalene or mono-alkylated phenol may be represented as follows:

$$\text{catalyst}$$
$$C_{10}H_7(C_mH_{(2m+1)}) + H_2 \text{ --------->} C_{10}H_{17}(C_mH_{(2m+1)})$$

$$\text{catalyst}$$
$$C_6H_4OH(C_mH_{(2m+1)}) + H_2 \text{ --------->} C_6H_{10}OH(C_mH_{(2m+1)})$$

Hydrogenation processes known to those skilled in the art may be used. A number of metal catalysts are suitable for promoting the hydrogenation reaction, including nickel, platinum, palladium, copper, and Raney nickel. These metals may be supported on a variety of porous materials such as kieselguhr, alumina, or charcoal. A particularly preferred catalyst for this hydrogenation is a nickel-copper-chromia catalyst described in US-A-3152998. Other known hydrogenation procedures are disclosed in US-A-4045508, -4013736, -3997622 and -3997621.

Unreacted monomer and phenol or naphthalene should be removed either before or after the hydrogenation step. Optionally, unreacted monomer and phenol or naphthalene may be stripped from the oligomerized alkylated product before hydrogenation and recycled to the catalyst bed for co-reaction. The removal or recycle of unreacted monomer and unreacted phenol or naphthalene, or, if after hydrogenation, the removal of non-oligomerized alkane and non-alkylated cyclohexanol or decahydronaphthalene, should be conducted under mild conditions using vacuum distillation procedures known to those skilled in the art. Distillation at temperatures exceeding 250°C may cause the oligomers to break down in some fashion and come off as volatiles. Preferably, therefore, the reboiler or pot temperature should be kept at or below 180°C. Procedures known by those skilled in the art to be alternatives to vacuum distillation also may be employed to separate unreacted components from the oligomer/alkylated phenol or alkylated naphthalene mixture.

While it is known to include a distillation step after the hydrogenation procedure to obtain products of various 100°C viscosities, it is preferred in the method of the present invention that no further distillation (beyond removal of any material that was not oligomerized or alkylated) is conducted. Thus, the method of this invention does not require the costly, customary distillation step, yet, surprisingly, produces a synthetic lubricant component that has excellent properties and that performs in a superior fashion. In some contexts, however, one skilled in the art may find subsequent distillation useful in the practice of this invention.

The invention will be further illustrated by the following examples:

## EXAMPLES

### Procedure

Olefin, phenol (or naphthalene), and clay catalyst were charged to a three-necked flask equipped with an overhead stirrer, thermometer, heating mantle, and water cooled condenser. A nitrogen purge was used. The mixture was vigorously stirred and heated to the desired temperature for the desired period of time. At the end of the reaction, the mixture was cooled to ambient temperature and filtered with suction.

In the Examples using naphthalene, unreacted olefin and naphthalene were removed by vacuum distillation until a pot temperature of 250°C was reached (0.8 KPa).

An autoclave was charged with nickel catalyst (5 wt.%) and the alkylated phenol (or alkylated naphthalene) and oligomer mixture prepared above. The autoclave was then sealed and flushed three or four times with hydrogen. The autoclave was then pressurized to 7MPa with hydrogen and heated to 200°C. The mixture was stirred at this temperature for 4.0 hours. The autoclave was repressurized to 13.9MPa (2000 psig) as needed. The mixture was then cooled to ambient temperature and filtered. Unreacted monomer was then removed from the filtrate under vacuum distillation (approx. 0.13KPa). The properties shown in the Tables below were determined on the "stripped" bottoms product.

Table 1 sets out the results obtained when olefins were oligomerized with phenol (Example 7 being provided for comprison).

Table 2 sets out the results obtained when olefins were oligomerized with naphthalene.

Table 3 sets out the properties of the hydrogenated products obtained according to Table 1.

Table 1

| Ex. No. | Reactants | (g) of Reactants | Harshaw/ Filtrol Catalyst | (g) of Catalyst | Time/Temp (Hr)/(°C) | Con.(%) of Olefin | Properties After Hydrogenation | | | |
|---|---|---|---|---|---|---|---|---|---|---|
| | | | | | | | % Remaining by TGA (250°C) | VIS (cSt) a· 100°C | VI | Pour Point (°C) |
| 1 | C-14A/Phenol | 360/ 40 | H/F Clay 123 | 40 | 5/160 | 85 | 96.1 | 9.47 | 104 | -34 |
| 2 | C-14A/Phenol | 320/ 80 | Dried H/F Clay 123 | 40 | 2/160 | 98 | 90.8 | 8.97 | 97 | -29 |
| 3 | C-151,181/Phenol | 320/ 80 | Dried H/F Clay 13 | 40 | 5/160 | 93 | 91.5 | 9.85 | * | -23 |
| 4 | C-14A,16A/Phenol | 320/ 80 | Dried H/F Clay 13 | 40 | 5/160 | 98 | 92.2 | 9.64 | * | -26 |
| 5 | C-151,181/Phenol | 320/ 80 | Dried H/F Clay 13 | 40 | 2/160 2/180 | 94 | 91.9 | 9.63 | * | -26 |
| 6 | C-14A16A/Phenol | 320/ 80 | Dried H/F Clay 13 | 40 | 2/160 2/180 | 97 | 93.0 | 9.71 | * | -23 |
| 7 | C-14A | 400 | H/F Clay 13 | 20 | 5/150 | 68 | 82.1 | 4.36 | 134 | -29 |

Con. = Conversion; A = Alpha; I = Internal; TGA = Thermogravimetric Analysis; VIS = Viscosity; VI = Viscosity Index; * = not determined.

EP 0 459 641 A2

TABLE. 2

| Ex. No. | Reactants | (g) of Reactants | Harshaw/ Filtrol Catalyst | (g) of Catalyst | Reactor | Time/Temp (Hr)/(°C) | Con.(%), Naphthln/ Olefin | Results from LC | | | D/T+ ratio |
|---|---|---|---|---|---|---|---|---|---|---|---|
| | | | | | | | | (M%) | D(%) | T(%) | |
| 8 | C-14A/Naphthalene | 80/ 20 | H/F Clay 113 | 10 | Flask | 6/160 | >95/75 | 25.0 | 49.5 | 46.9 | 1.06 |
| 9 | C-14A/Naphthalene | 320/ 80 | H/F Clay 113 | 40 | Flask | 5/160 | 98/75 | 25.0 | 41.6 | 55.0 | 0.76 |
| 10 | C-14A/Naphthalene | 360/ 40 | H/F Clay 124 | 40 | Flask | 6/160 | */92.3 | 7.76 | 28.8 | 63.6 | 0.45 |
| 11 | C-14A/Naphthalene | 380/ 20 | H/F Clay 124 | 40 | Flask | 6/160 | */88.8 | 11.2 | 36.1 | 52.7 | 0.69 |
| 12 | C-14A/Naphthalene | 320/ 80 | H/F Clay 113 | 40 | Flask | 6/160 | */87.7 | 18.4 | 46.0 | 35.6 | 1.29 |
| 13 | C-14A/Naphthalene | 320/ 80 | H/F Clay 113 | 40 | Flask | 6/160 | */86.0 | 14.0 | 36.5 | 49.5 | 0.74 |
| 14 | C-14A/Naphthalene | 320/ 80 | H/F Clay 113 | 20 | Flask | 6/160 | */80.6 | 19.4 | 45.1 | 35.5 | 1.27 |
| 15 | C-14,16A/Naphthln | 320/ 80 | H/F Clay 13 | 40 | Flask | 6/160 | */84.0 | 16.0 | 38.2 | 45.9 | 0.83 |
| 16 | C-13,14I/Naphthln | 320/ 80 | H/F Clay 13 | 40 | Flask | 6/160 | */85.2 | 14.8 | 37.4 | 47.8 | 0.78 |
| 17 | C-15,18I/Naphthln | 320/ 80 | H/F Clay 13 | 40 | Flask | 6/160 | */71.0 | 29.0 | 43.8 | 27.1 | 1.62 |
| 18 | C-14A/Naphthalene | 320/ 80 | Dried H/F Clay 124 | 40 | Flask | 5/140 | */70.4 | 39.6 | 41.2 | 29.2 | 1.41 |
| 19 | C-14A/Naphthalene | 320/ 80 | Dried H/F Clay 124 | 40 | Flask | 4/180 | */82.2 | 17.8 | 40.0 | 42.2 | 0.95 |
| 20 | C-14A/Naphthalene | 396/ 4 | Dried H/F Clay 124 | 40 | Flask | 5/160 | */71.4 | 28.6 | 42.4 | 29.0 | 1.46 |

Con. = Conversion; M = Monomer; D = Dimer; T+ = Trimer+Tetramer+Pentamer,etc.; A = Alpha; I = Internal; * = not determined.

Table 3

| Ex. No. | Mixture Prepared as Recorded in Table I | Properties Before/After Hydrogenation | | | |
|---------|------------------------------------------|---------------------------------------|----------------|----------|------------------------|
| | | % Remaining by TGA | Vis(cSt) at 99°C | VI | Pour Point (°C) |
| 8 | C-14A Olig/Alkyl Nap | 94.8/ 93.2 | 12.3/ 12.8 | 110/ 110 | -34/-37 |
| 9 | C-14A Olig/Alkyl Nap | 93.5/ 93.5 | 10.3/ 10.5 | 120/ 120 | -32/-37 |
| 10 | C-14A Olig/Alkyl Nap | 89.2/ 89.2 | 7.61/ 7.92 | 143/ 121 | -34/-37 |
| 11 | C-14A Olig/Alkyl Nap | * | 4.76/ 5.04 | 133/ 126 | -32/-34 |
| 12 | C-14A Olig/Alkyl Nap | * | 13.3/ 13.4 | 111/ 106 | -32/-37 |
| 13 | C-14A Olig/Alkyl Nap | 95.8/ 95.8 | 12.9/ 12.4 | 110/ 105 | -34/-37 |
| 14 | C-14A Olig/Alkyl Nap | 97.2/ * | 9.84/ 10.1 | 108/ 106 | -34/-37 |
| 15 | C-14,16A Olig/Alkyl Nap | 86.0/ * | 12.8/ 13.1 | 110/ 112 | -29/-40 |
| 16 | C-13,14I Olig/Alkyl Nap | 96.4/ * | 12.4/ 12.8 | 106/ 104 | -40/-34 |
| 17 | C-15,18I Olig/Alkyl Nap | 96.6/ * | 11.7/ 12.3 | 107/ 108 | -26/<-46 |
| 18 | C-14A Olig/Alkyl Nap | 96.2/ * | 9.06/ 9.69 | 114/ 114 | -32/-32 |
| 19 | C-14A Olig/Alkyl Nap | * | 11.1/ 12.1 | 106/ 110 | -32/-37 |
| 20 | C-14A Olig/Alkyl Nap | * | 5.59/ * | 139/ * | -29/* |

A=Alpha; I=Internal; Alkyl Nap
= Alkylated Naphthalenes; Olig = Oligomers; * = not determined.

## Claims

1. A synthetic lubricant base stock comprising reduced oligomers prepared from an olefin composition comprising a linear olefin having from 10 to 24 carbon atoms characterized in that it additionally contains an alkyl-substituted cyclohexanol or alkyl-substituted decahydronaphthalene, whose alkyl groups have 10 to 24 carbon atoms.

2. A synthetic lubricant base stock according to Claim 1, characterized in that it contains from 1 to 80 wt.% of alkyl-substituted cyclohexanol or alkyl-substituted decahydronaphthalene.

3. A synthetic lubricant base stock according to Claim 1 or 2 characterized in that the linear olefin has 12 to 18 carbon atoms; and the alkyl groups of the alkyl-substituted cyclohexanol or alkyl-substituted decahydronaphthalene have from 12 to 18 carbon atoms.

4. A synthetic lubricant base stock according to any one of Claims 1 to 3 characterized in that the linear olefins comprise alpha-olefins internal olefins or a mixture thereof.

5. A synthetic lubricant base stock according to any one of Claims 1 to 4 characterized in that the olefin composition comprises said linear olefins and up to 20 weight % of propylene.

6. A synthetic lubricant base stock according to any one of Claims 1 to 4 characterized in that the olefin composition comprises said linear olefins and up to 20 weight % of 1,3-diisopropenyl benzene or alpha-methyl styrene.

7. A synthetic lubricant base stock according to any one of Claims 1 to 4 characterized in that the olefin composition comprises said linear olefins and a vinylidene olefin having 10 to 24 carbon atoms.

8. A synthetic lubricant base stock according to Claim 7 characterized in that the olefin composition contains 5 to 40 weight % of the vinylidene olefin.

9. A process for preparing a synthetic lubricant base stock according to any one of Claims 1 to 8 comprising contacting the olefin composition with an acidic montmorillonite clay and hydrogenating the resulting oligomer mixture characterized in that phenol or naphthalene is co-reacted with the olefin composition.

10. A process according to Claim 9 characterized in that the phenol or naphthalene, and the olefin composition are co-reacted in a weight ratio of 1:99 to 2:3.

11. A process according to Claim 9 or 10 characterized in that the clay is activated with a Lewis acid.